# EUROPEAN PATENT SPECIFICATION

(11) **EP 3 232 978 B1**
(45) Date of publication and mention of the grant of the patent: **05.02.2020**
(21) Application number: 15817793.1
(22) Date of filing: 16.12.2015
(51) Int. Cl.: A61B 90/11, A61B 90/16, A61B 90/00

(54) **INTERVENTION GUIDANCE DEVICE**
INTERVENTIONSFÜHRUNGSVORRICHTUNG
DISPOSITIF DE GUIDAGE D'INTERVENTION

(30) Priority: 18.12.2014 GB 201422551
(43) Date of publication of application: 25.10.2017
(73) Proprietor: Norwegian University of Science and Technology (NTNU), 7491 Trondheim (NO)
(72) Inventor: BRATBAK, Daniel Fossum, 7023 Trondheim (NO)
(74) Representative: Dehns
(86) International application number: PCT/EP2015/079989
(87) International publication number: WO 2016/096984

(56) References cited:
- WO-A2-2006/118915
- US-A1- 2006 281 991
- US-A1- 2007 250 105
- US-A1- 2012 316 486

## Description

The invention relates to a guidance device for guiding surgical interventions within the head, for example for guidance of an injection of a substance into a target site in the head. In one example the device is used for guidance of injections towards cranial parasympathetic ganglia. The disclosure also relates to the use of such a device in the treatment of medical conditions, for example in the treatment of primary headaches.

Migraine is a primary headache that may be characterized as a unilateral headache associated with symptoms like nausea, photophobia and phonophobia. More than 50% have as well cranial autonomic symptoms such as lacrimation, conjunctival injection, nasal congestion and rhinorrhoea.

A possible mechanism for a migraine attack is parasympathetic activation with nitrogen oxide (NO) as transmitter inducing dilatation of cranial blood vessels, plasma protein extravasation and release of inflammatory substances. The catalysing enzyme for NO, NOS (NO synthases), has been located in perivascular nerve fibres on cerebral arteries and traced back to the sphenopalatine ganglion (SPG) and otic ganglion (OG), as described by Olesen J. in "The role of nitric oxide (NO) in migraine, tension-type headache and cluster headache", Pharmacology and Therapeutics, 2008;120;157-171.

Blocking of the SPG by application of lidocaine has shown to be effective in randomised, controlled studies of acute treatment of migraine (see Maizels M, Scott B, Cohen W and Chen W, "Intranasal lidocaine for treatment of migraine: a randomized, double-blind, controlled trial" JAMA, 1996;276(4):319-21 and Maizels M and Geiger AM, "Intranasal lidocaine for migraine: a randomized trial and open-label follow-up", Headache, 1999;39(8):543-51). Blocking via botulinum toxin is also described in the prior art, for example in US 7981433.

The trigeminal autonomic cephalalgias (TACs) are a group of primary headache disorders characterized by unilateral head pain that occurs in association with ipsilateral cranial autonomic features such as lacrimation, conjuctival injection and nasal symptoms. The TACs include hemicrania continua, paroxysmal hemicrania, short lasting unilateral neuralgiform headache with conjunctival injection and tearing /cranial autonomic features (SUNCT/SUNA) and cluster headache.

Cluster headache is a severe unilateral headache associated with ipsilateral autonomic symptoms and characterised by a circannual and circadian periodicity (see Goadsby PJ, Cittadini E, Burns B and Cohen A, "Trigeminal autonomic cephalalgias: diagnostic and therapeutic developments" Curr Opin Neurol, 2008;21:323-330). Approximately 90% suffer from the episodic form and 10% from the chronic form. Based on functional neuroimaging central to the pathophysiology of the disease may be an abnormality in hypothalamic function that facilitate a cascade of metabolic and other biochemical events triggering an attack (see Cohen AS and Goadsby PJ, "Functional neuroimaging of primary headache disorders" Expert Rev Neurother, 2006;6(8):1159-1171). This sets off a positive feedback system involving the trigeminovascular system as the afferent limb and the parasympathetic outflow from the superior salivatory nucleus via the facial nerve through the SPG and OG as the efferent limb (see Goadsby PJ, "Pathophysiology of cluster headache: a trigeminal autonomic cephalgia" Lancet Neurol. 2002;1:251-57). Thus, vasodilatation of the pain-producing large cranial vessels and dura mater starts a reflex activation of parasympathetic vasodilator efferents which activate the trigeminal endings further to produce the excruciating pain and the parasympathetic symptoms (lacrimation and nasal congestion/secretion) seen in cluster headaches. In addition, the carotid swelling leads to a neuropraxic lesion of the sympathetic plexus surrounding the artery, resulting in a partial ipsilateral Horner's syndrome (ptose, miosis and conjunctival injection).

Current strategies for surgical treatment of these headaches include neurodestructive procedures targeting the trigeminal system (afferent limb) and the SPG (efferent limb), and neurostimulating procedures targeting the great occipital nerve and grey matter of hypothalamus (deep brain stimulation, DBS). Thus, cranial autonomic ganglia, and especially SPG and OG, are thought to have a role in the development of primary headaches and treatments have been established targeting the SPG.

Primary headaches may be hard to treat and the need for preventive treatments is enormous. Apart from CGRP antagonism, inhibition of the NO pathway may be considered the best documented and most promising target for treatment of primary headache (as described by Olesen J. in the reference above).

The trigeminal nerve is involved in all types of headache, including secondary headaches, i.e. headaches caused by other pathologies.

Sinonasal polyposis is a chronic hyperplastic disease of the nasal mucosa and the paranasal sinuses. There is a well established association between polyposis and rhinitis. The causes underlying the association could be due to chronic inflammation most likely induced by unstable autonomous nerve control of nasal vasomotor activity. This may precede the occurrence of nasal polyps. Vasomotor rhinitis seems to be related to an imbalance in the cranial autonomic system between parasympathetic and sympathetic activity. Therapies include vidianectomi and other forms of autonomic denervation which blocks parasympathetic activity through the SPG. Vidianectomi and other forms of autonomic denervation have also been an option for treating allergic rhinitis and new modified surgical techniques yield optimistic results.

Blocking the parasympathetic activity passing through the SPG by vidian neurectomy has shown to be effective in allergic rhinitis (see Wan-Fu SU, Shao-Cheng Liu, Feng-Shiang Chiu and Chia-Hsuan Lee. Antegrade transsphenoidal vidian neurectomy: Short-term surgical outcome analysis. Am J Rhinol Allergy 2011;25:e217-e220), vasomotor rhinitis and rhinosinusitis with polyposis (see Cassano M, Mariano G, Russo L, Cassano P. Sphenopalatine artery ligation with nerve resection in patients with vasomotor rhinitis and polyposis: a prospective, randomized, double-blind investigation. Acta Oto-Laryngologica 2012;132(5):525-32).

Almost all patients who undergo parotidectomy will to some extent develop Frey syndrome (auriculotemporal syndrome or gustatory sweating) after surgery, because of aberrant regeneration of cut parasympathetic fibres between otic ganglion and subcutaneous vessels. Frey syndrome may also occur after extirpation of the submandibular gland, mandibular condylar fracture, and obstetric trauma caused by forceps. Nontraumatic causes are sympathectomy, autonomic neuropathy in diabetes mellitus, herpes zoster infection, and metabolic diseases. Frey syndrome may cause considerable social embarrassment and social incapacity due to profuse flushing and sweating when eating. Blocking the parasympathetic activity through the OG may constitute an effective treatment for these patients.

The cranial autonomic ganglia, and especially the SPG and the OG, are hence interesting targets for treating such entities, but they are not easily reached for interventions such as infiltration with pharmacological substances, destructive procedures or neuromodulation.

There are four paired cranial parasympathetic ganglia: sphenopalatine (pterygopalatine) ganglion (SPG), otic ganglion (OG), ciliary ganglion, and submandibular ganglion.

The SPG is pyramid shaped with a mean diameter of 3.5 mm. It is suspended from the maxillary nerve by the sphenopalatine nerves. Preganglionic parasympathetic fibres form the nervus intermedius of the facial nerve synapse with postganglionic fibres innervating the lacrimal gland, mucosa of the sinonasal cavity and cerebral blood vessels. Postganglionic sympathetic fibres from the superior cervical ganglion pass through the ganglion as well as sensory nerves from the maxillary nerve that innervates the palate and the epipharynx. The SPG can be identified using MRI.

The SPG is situated in the sphenopalatine (pterygopalatine) fossa (SF) and has the shape of a funnel flattened in the coronal plane. It is wider superiorly and then narrows down inferiorly with the apex pointing downwards into the greater palatine canal. SF has the following boundaries; superiorly with the infraorbital fissure, laterally with the pterygomaxillary fissure, medially with the palatine bone, posteriorly with the pterygoid plates, anteriorly with the posterior wall of the maxillary sinus and inferiorly with the palatine canal. Additionally, it communicates with the nasal cavity through the sphenopalatine foramen and the middle cranial fossa through the vidian canal and foramen rotundum. It can be divided in three compartments, an anterior compartment containing mainly blood vessels, a middle compartment containing mainly adipose tissue, and a posterior compartment containing mainly neural structures.

The maxillary artery enters the SF through the pterygomaxillary fissure and branches into the sphenopalatine artery, descending palatine artery, infraorbital artery, alveolar arteries and the artery of the pterygoid canal. The SF is often devoid of endoscopic identifiable veins. Blood vessels of the SF are tightly packed as they loop the anterior compartment and therefore a lateromedial intervention is more likely to cause a bleeding than an anteroposterior approach.

The average distance from the SPG to the vidian canal is 2.7 mm, to the infraorbital fissure 20.3 mm and to foramen rotundum 4.7 mm. It is normally located in the same vertical and horizontal plane as the vidian canal and posteriorly for the sphenopalatine foramen. The sphenopalatine foramen is vertically orientated located in the superomedial corner of SF with a diameter of 5-6 mm and typically located below the posterior end of the line of attachment of the middle turbinate and crista ethmoidalis, but this may vary. The average distance from the piriform aperture is 48 mm with an angle of elevation from the nasal floor is 22 degrees.

Such information of the distances from SPG to landmark identifiable on CT may be used to mark the SPG for image-guided interventions when MRI is contraindicated or not available.

The oral cavity communicates with the sphenopalatine fossa through the greater traspalatinal canal. The inferior opening is situated on the medial side of the second molar and the length of the canal is on average 25 mm. The canal transmits the descending palatine artery and vein, and the greater and lesser palatine nerves.

The OG is an oval structure measuring approximately 4 mm x 3 mm x 1.5 mm. It is composed of parasympathetic fibres arising in the inferior salivatory nucleus in the medulla, sympathetic fibres form the superior cervical sympathetic ganglion, and motor fibres from the mandibular branch of the trigeminal nerve. The OG supplies secretory fibres to the parotid gland and parasympathetic fibre to cerebral blood vessels. It is situated just posterior of the lateral pterygoid plate below the foramen ovale in the infratemporal fossa and adjacent to the middle meningeal artery, mandibular nerve and buccal nerve.

For minimally invasive interventions in the SF there are three surgical approaches, each with its advantages and disadvantages; a lateral approach through the pterygomaxillary fissure, a medial transnasal approach through the sphenopalatine foramen and a transoral approach through the greater palatine canal. All approaches give a relatively easy access to SF for someone skilled to the art, but there are pivotal differences if a high-precision intervention in the closest proximity of the SPG is needed.

Image guided surgery (IGS) was developed to improve accuracy and precision. Such technology is used to assist in orientation by displaying the position of a pointer or surgical instrument on a medical image. Armless systems may be based on light, sound waves or magnetic fields. With the use of a computer platform, a tracking system and a body marker, a pointer or other instrument can be calibrated so that the navigation system will display the tip of the instrument correctly. The instruments are calibrated in advance by the manufacturer or the surgeon may use a universal instrument integration system to calibrate basically any instrument. This system is based on a set of universal clamps attached to the instrument. There are several limitations to this solution. Firstly, attaching the clamps can be challenging and they can easily move, hence giving a wrong impression of the actual localization of the instrument on the medical image. Secondly, semi-rigid instruments are not suitable for calibration because they can bend after calibration, such as e.g. a thin needle or a long forceps.

The lateral approach is typically carried out under local anaesthesia. Typically a high-precision intervention would be an lateral approach. Using the lateral approach there is a straight line through soft tissue from the skin to the SF, SPG, orbita and the sphenopalatine foramen. The distance from the skin to the SF or the SPG is approximately 6 cm making it next to impossible to achieve a high precision infiltration without the use of IGS. Violating the sphenopalatine foramen could result in a complicated posterior epistaxis, violating the infraorbital fissure could damage intraorbital tissue. Using the suprazygomatic approach, which is described in US 7981433, for example, the sphenoid bone will normally obstruct access to the SF and in particular the middle and the posterior compartment and almost always obstruct access to the SPG, making it quite safe, but not applicable for high-precision interventions. If anatomical variations enable advancing a needle to the close proximity of the SPG by a suprazygomatic approach, it would be next to impossible to successfully target such a small structure with a conventional injection technique as described in US 7981433. Due to the low diffusion rate of botulinum toxin and the fact that the SF mainly contains adipose tissue, a hydrophilic substance injected using these techniques will rarely reach its target.

The medial transnasal approach is difficult to perform under local anaesthesia due to the sensible posterior region of the nasal cavity, and the use of general anaesthesia makes it much less accessible. Due to the complex sinonasal anatomy it is normally performed by a rhinologist. For someone skilled in the art this approach may be the most accurate, mainly due to the low distance between the puncture site and the SPG. Normally such an approach is done by advancing the needle through the sphenopalatine foramen, risking damage to the sphenopalatine artery/arteries. The palatine bone, which constitutes the anterior border of the sphenopalatine foramen, is quite thin, and a suitable needle can quite easily be advanced through the bone, avoiding possible damage to the sphenopalatine artery.

However, such a procedure can easily bend the needle used, which will generally be an 18G needle or thinner. After it has been advanced through the bone the end of the needle is in the soft tissue and there is no way to know if deformation has occurred or to what extent, making the intervention unsafe and imprecise, with the use of IGS or not. For injections in deep tissue a 25G needle or thinner is recommended to avoid unnecessary tissue damage, including bleedings and nerve damage. Furthermore, the thicker the needle the bigger the dead space, which hinders use of small injection volumes. As a consequence of these issues, needles suitable for SPG injection using the medial approach and also other approaches are not suitable for high-precision injections.

The transoral approach can be done with local anaesthesia. However, due to the direction of the palatine canal towards the very anterior part of the SF, high-precision interventions targeting the SPG are currently not feasible with this approach.

Intervention targeting the OG can be done via a lateral approach as described in interventions targeting the trigeminal ganglion through the oval foramen, or lateral approaches with the same injection sites as described above, i.e. lateral or suprazygomatic. It is also possible to apply a transnasal medial approach through the maxillary ostium and the posterior wall of the maxillary sinus and advancing adjacent to the lateral pterygoid plate. With this transnasal medial approach one can avoid important nerves and blood vessels in the infratemporal fossa and was performed without complications or side effects. This medial approach seems as well appropriate for neurostimulators as it can be situated and anchored to the pterygoid plate.

The cranial parasympathetic ganglia including the SPG and OG are surrounded by critical neural structures and organs like e.g. brain and eyes. Drug impact of these structures can cause serious complications and should be avoided. In addition, some medications diffuse slowly and they must be injected with millimetre accuracy to reach their target. As a result, accuracy is important in various situations:
1) When using a drug or implant that only works exactly where it is injected/situated.
2) Use of a diffusible drug that must be injected at a safe distance from sensitive structures (e.g. brain or eye).
3) When using a drug or implant that can cause serious complications if it is injected accidentally in the wrong place.
4) For injection into an area where the needle can damage other nearby structures.

All four factors are important when it comes to injections of botulinum toxins (as known by the trade name Botox, for example) or similar neurotoxins to the SPG or OG, and some or all of the factors also apply to other medications that one can envisage using in blocking of cranial parasympathetic ganglia. Moreover, since the same or similar requirements arise in many other situations requiring delivery of a substance or insertion of an instrument to a targeted site within the human or animal body then a device and/or method capable of addressing the need for targeting of the cranial parasympathetic ganglia will have numerous other uses and advantages.

As noted above, prior art such as US 7981433 discloses administration (topical and by injections) of neurotoxins (e.g. Botox) to parasympathetic (including SPG), trigeminal and occipital nerves in the treatment of headaches, amongst other things.

US 7981433 describes an injection technique, specifically a lateral approach, which is a conventional suprazygomatic approach. This approach makes it impossible to accurately deposit substances, since the sphenoid bone will normally obstruct access to the SF and in particular the middle and the posterior compartment and almost always obstruct access to the SPG, making it quite safe, but not applicable for high-precision interventions. Due to the low diffusion rate of botulinum toxin and that the SF mainly contains adipose tissue, a hydrophilic substance will rarely reach its target. There is no consideration in US 7981433 of the techniques required to reach other parasympathetic ganglia (most importantly the OG). Thus, there is a significant unmet need for a safe, high-precision system for targeting of cranial parasympathetic ganglia and other similar target sites in the human or animal body.

Image guided techniques are quite complex, especially when performed on awake patient and can move. The surgeon must then compensate continually for the movement of the patient. For procedures performed under sedation and general anaesthesia, the head of the patient can be fixed during a procedure, e.g. by screws to the cranium. For conscious patient this is very painful, and sometimes more importantly an uncomfortable experience, especially combined with a procedure performed towards the head/face that causes pain.

US 2007/250105 A1 discloses a method of treating a constricted sinus passageway. The method includes traversing the canine fossa region of the patient to form a sinus cavity. An elongate member is then inserted through the passageway. The elongate member comprises an inflation member that is positioned within the passageway, and is subsequently inflated to expand a portion of the passageway.

Viewed from a first aspect, the invention provides a guidance device for guidance of surgical interventions on a patient, the surgical interventions requiring an intervention device to surgically enter the body and be directed through body tissues within the mouth to a target site within the patient's head, the guidance device comprising: a hollow tube for guiding the intervention device into the body and directing it to the target site within the patient's head; a mouthpiece arranged to anchor the device in a fixed orientation relative to the patient's upper jaw or lower jaw; and a targeted mounting supporting the hollow tube on the mouthpiece, the mounting being tailor-made for the patient based on the patient's anatomy and for directing the hollow tube in a desired orientation relative to the mouthpiece to thereby direct the intervention device through body tissues within the mouth to the target site in the patient's head via a transpalatine approach.

The target site may be any location that does not move relative to the patient's upper jaw, or the patient's lower jaw, as the case may be. It should be noted that the first aspect relates to a device for medical surgery and for surgical interventions on the body involving insertion of an intervention device through body tissue to reach a target site. The device of the first aspect is hence not suitable for, or intended for, use in dental surgery and similar procedures involving surgery on the teeth. The device of the first aspect may be for procedures excluding dental procedures, and thus the device may be for procedures excluding operations on the teeth, operations for placing dental implants and so on. In example embodiments the device is for targeted surgical procedures involving surgically entering the body with an intervention device through tissue within the mouth.

By having a mouthpiece that anchors the device at the upper jaw or the lower jaw the invention provides a stable support for the guidance device, such that the desired orientation remains fixed relative to the target site even when the patient moves. Since the guidance device and hence the intervention device can be secured to a structure that does not move relatively in relation to the target site, e.g. the sphenopalatine ganglion with fixation to the upper jaw, then fixation of the head will not be necessary. The upper jaw is fixed relative to most structures of the head/face, with the exception of the lower jaw and structures that move with movements of the lower jaw.

The use of this device to guide an intervention device for high-precision image guided procedures will make the performance of such procedures much easier. Procedures done today by highly specialized and trained surgeons will be available for wide range of medical specialists, including physicians and general practitioners, making the procedures depending on such techniques much more available and in the hand of the diagnostic physician, making treatments easier to implement. Patient care can hence be improved.

In some examples the mouthpiece is arranged to be anchored in a fixed orientation relative to the upper jaw. It is preferred for the mouthpiece to be arranged to be mounted to the patient's upper teeth in order to thereby anchor the device to the upper jaw. The mouthpiece may alternatively or additionally be mounted to the upper gums and/or the roof of the mouth.

In other examples the mouthpiece is arranged to be anchored in a fixed orientation relative to the lower jaw. In this case the mouthpiece may be arranged to be mounted to the patient's lower teeth.

The mouthpiece could optionally be custom made for each patient, or a generic mouthpiece may be used in conjunction with a mouldable inner material for conforming to the patient's upper or lower teeth, gums and/or roof of the mouth. The mouldable inner material may be arranged to set once it has been pressed into the patient's mouth in order to provide a strongly fixed anchoring to the jaw. The mouthpiece may be moulded and fitted to the patient by means of known techniques, such as techniques used in the dental industry. The mouthpiece may be fixed by a mechanical fixing such as a clamp.

One example mouthpiece may be made of heat-sensitive acrylic material, which may be in a gum shield type shape. Another example mouthpiece is ResMed Narval CC.

Advantageously, the device can be a single-use device intended to be disposed of after use. The device can hence be supplied in sterile packaging and used without any special preparation/sterilisation being required, before being disposed of after surgery. Although the device is designed for use with complex and extensive guided surgery systems, the design of the device does not require any expensive materials or moving parts and it can be made disposable without any disadvantage in relation to costs.

The inside of the mouthpiece may be covered with plastic compound, gel or similar to aid good fixation. Adhesive compositions known for use with dentistry may be used.

Optionally, the mouthpiece may be provided with a strap for extending around the patient's head to secure fixation and further hinder movement of the device. This is particularly useful for a mouthpiece secured to the upper jaw, since this does not move relative to the major portion of the patient's head.

The hollow tube is attached to the mouthpiece via the mounting.

The mounting is a targeted mounting that is tailor-made for each patient. For example the mounting may be manufactured for a specific target site based on the patient's anatomy. The patient's bone and soft tissue structure may be mapped using imaging techniques, which then allows determination of a required location and orientation for the mounting and for the hollow tube held by the mounting. This arrangement might be used for target sites in the patient's head that are approached from the inside of the mouth. A tailored mouthpiece can be used to target any structure, such as the SPG or OG from a device anchored to the patient's upper jaw as mentioned above.

One example device has a patient tailored mounting arranged for guiding the intervention device toward the SPG via a transpalatine approach, and hence the mounting may be arranged to direct the hollow tube toward the inferior opening of the palatine canal. This provides great assistance to the interventionist in accurately targeting the SPG (or other target site) via the transpalatine approach. The penetration depth of the device may be calculated form the medical images, and the length of the device or of the hollow tube adjusted accordingly so that an accurate placement of the device is enabled. A trackable needle might be used as the intervention device, as discussed below.

With this type of mounting the guidance device may be arranged to receive the intervention device at the front (by the frontal teeth) and to direct the intervention device to exit within the patient's mouth at a required orientation, at the inferior opening of the palatine canal, with the right inclination to facilitate the advancement of the intervention device into the canal.

The hollow tube may have a varying construction depending on the intervention device that will be used. Preferably the hollow tube is arranged to support an intervention device, such as an injection device, and to direct it in a required orientation. In one example the hollow tube, takes the form of a lumen, which can hence guide a smaller lumen, catheter, needle or similar. The hollow tube can be targeted with a required position and angulation relative to the mouthpiece by means of, a patient specific targeted mounting as described above. The hollow tube can then direct the intervention device in the desired manner, for example by aligning a needle or other injection device with a transpalatine approach to the target site.

The correct orientation of the hollow tube may be determined based on imaging techniques. This could be done manually by a skilled operator, but it is preferred to have a computer guided device. Hence, in a preferred embodiment the guidance device incorporates a navigational array or an anchor for a navigational array. The navigational array may take the form of a localiser. The navigational array may be arranged for use with a surgical navigation system that may be optical, electromagnetic or any other system. This enables the user to accurately align the hollow tube with the desired orientation in order that the guidance device can direct an intervention device towards the target site. The navigational array may be integrated in the mouthpiece. In this case an additional localiser to register the patient will not be necessary. After calibrating the device for an image guided system, it is beneficial to ensure that the device cannot move relative to the patient localizer without the movement being detected.

Advantageously the device may include an indicator for providing a warning of undesirable movement. In one preferred arrangement the guidance device comprises a light source for pointing at the patient. The light source should be attached to the mouth piece and able to be locked in place relative to the hollow tube. When the device is calibrated, the light source may be pointed towards a specific anatomical landmark or a mark on the skin made with a standard marker. This means that any inadvertent movement of the hollow tube relative to the target site will cause the light to move away from the anatomical landmark or the mark on the skin, making the interventionist aware of the incident. In addition or alternatively, the device may have a camera attached that can register an inadvertent event, e.g. by videometry or simply using a marker registered by the camera, e.g. a fluorescent marker, or illumination from the light source, if present.

The device may incorporate an additional patient localiser mounted on the patient's head, for example via a headband. In this case, with a patient localiser on the patient's head and a navigation array mounted to the mouthpiece then the navigation system may detect any relative movement between the patient localiser and mouthpiece and warn the interventionist. In an alternative arrangement there may be a patient localiser with a light source that can be directed towards the mouthpiece, and the mouthpiece can have a target in place of a navigational array. When the patient is registered the light can be targeted on the mouthpiece and if the light moves away from the target during the procedure this acts as a warning of patient movement. The target may amplify the light to be easier to detect, for example the target may be a prism and/or may have a fluorescent element. The target may also carry a sensor that will warn the interventionist by setting off an alarm if the light does not hit the target. It will of course be understood that the light source and target or sensor could be reversed, with the light source at the mouthpiece and the target on the patient. Furthermore, the use of light is not the only option and other forms of radiation source and sensor may be used.

The guidance device may comprise a small screen. Images from the navigation system could then be sent to the screen for use in aligning the hollow tube with the desired orientation. Movements registered on the navigation system will then be aligned with movement of the device and working instruments, making it easier for a physician without special training performing such procedure. Such screen device may also carry a camera for use as described above.

The guidance device may be used in combination with known intervention devices such as, for example, the device described in WO 2014/037524. It is also possible to make effective use of more basic intervention devices, for example devices not provided with an integrated navigational array or the like, since the guidance device will ensure that the intervention device approaches the patient at the correct angulation for the desired procedure.

The navigation array may comprise optical markers or electromagnetic location sensors, for example optical reflectors such as reflector balls or electromagnetic coils. Any suitable navigation array system can be used. The navigation array may comprise a plurality of markers located in plane with one another and at known locations relative to the end piece. In one preferred embodiment there are at least three markers, for example there may be four or five markers. The navigation array should be rigidly connected to the hollow tube. The hollow tube may have a known orientation and size relative to the navigation array, or a calibration sequence may be performed to provide appropriate data concerning the orientation and size of the hollow tube relative to the navigation array. A rigid and integrated connection of the navigation array with the hollow tube is preferred since this provides the least risk of inaccuracy and in advertent misalignment of the navigation system with the hollow tube.

Alternatively, when an anchor point for a navigation array is provided then the anchor point should be arranged for rigid connection of the navigation array to the hollow tube. The anchor point may, for example, be for connection to an array of the type supplied under the trade names SureTrack® Universal tracker from Medtronic and Brainlab Instrument Adapter System from Brainlab.

The guidance device may comprise a proximal piece for holding a proximal end of an intervention device used with the guidance device. The proximal piece may be arranged to allow for measurement of and/or control of the depth of insertion of an intervention device into the patient. The proximal piece may be positioned at a proximal end of the hollow tube. It is preferred for the proximal piece to comprise parts that are moveable relative to the hollow tube and are for fixed connection to the intervention device. Such parts can be used in the manipulation of the intervention device as described below.

In a particularly preferred embodiment the proximal piece comprises one or more clamp(s) for attachment of the intervention device. A clamp or clamps may advantageously be provided on the proximal piece to releasably fix the intervention device in place relative to the hollow tube.

When the intervention device has been inserted into the body, guided by the guidance device, to a suitable point with reference to a target site then the intervention device can be operated by manipulation of the proximal end of the intervention device at the proximal piece. For example, the intervention device may be extended from the distal end of the hollow tube to move it closer to the target site. When the intervention device includes a needle this allows for highly accurate targeted injection without the risk of damaging the target site. A scale is preferably provided on the proximal piece in order to show the movement of the intervention device, for example how far the intervention device has been inserted into the body.

The proximal piece may comprise two clamps for releasable connection to the intervention device, with one clamp slidable relative to the scale and hence useable to indicate movement of the intervention device. Alternatively, or in addition, the proximal piece may comprise positional markers, e.g. in the case of an optical system, reflectors, for indicating the distance. For example, a positional marker may slide along the proximal piece connected to an associated one of the clamps, which in turn may be for fixed connection to the intervention device during use, so that the positional marker moves along with the intervention device. In a preferred embodiment the proximal piece includes a handle, such as a ring piece, for enabling the user to push or pull the intervention device with the thumb or a finger.

The moveable parts of the proximal piece, which are for connection to the intervention device, may advantageously be connected to the navigation array, or to a further navigational array. This may allow for computer guidance of the depth of insertion of the intervention device.

Advantageously, the guidance device can be used in relation to a target site at any region of the patient's skull that is fixed relative to the upper jaw or the lower jaw, as the case may be. In some preferred embodiments, with the device fixed to the upper jaw, the guidance device is for targeting of the SPG or other of the cranial parasympathetic ganglia, for example the OG. The guidance device may hence be arranged for use with a lumen and/or needle arrangement capable of advancing along the transpalatine approach.

The intervention device may be included with the guidance device in order to form a system for guided surgical interventions. The intervention device may include a navigation array, such as a localiser, in order to allow for guided placement of the intervention device, such as guided insertion to a required depth. The intervention device may for example be a needle within a lumen, or it may be a neurostimulator such as a neuromodulator. The intervention device may comprise an implant such as a steroid implant or a drug eluting stent. Steroid implants and drug eluting stents are used for treating sinusitis, and postoperatively to avoid recurrence. Such devices are normally implanted in the ethmoidal sinuses. The implantation technique may in certain cases be quite difficult due to local anatomy. It is described in the literature serious complications due to unintentional insertion in the orbit. Such implants may be implanted with guidance from the device described herein in a safer manner than the prior art techniques.

In some preferred examples the intervention device is a trackable needle, for example an electromagnetic needle, that allows for the operator to track the location of the needle within the body, and in particular a needle that allows the operator to track the location of the needle tip within the body. Such a system can provide additional accuracy in relation to determining when the needle has reached the target site. Possible devices include those marketed under the trade name Aurora by Northern Digital, Inc (NDI) of Ontario, Canada.

The intervention device may also be a needle for core needle biopsy, a needle for fine needle biopsy, an electrode for electric or radiofrequency ablation therapy or a cannula for chemical ablative therapy.

The device may be arranged for use in the method described below in the second aspect and preferred/optional features thereof.

Viewed from a second aspect, the invention provides a method of targeting an intervention device for later surgical intervention on the body by use of a guidance device, where the guidance device comprises a hollow tube for guiding an intervention device; a mouthpiece arranged to anchor the device in a fixed orientation relative to the patient's upper jaw or lower jaw; and a targeted mounting supporting the hollow tube on the mouthpiece, the method comprising: non-surgically attaching the mouthpiece of the guidance device to the patient's upper jaw; determining a desired orientation for a the hollow tube of the guidance device which will enable the hollow tube to later guide the intervention device to surgically enter the body and be directed through body tissues within the mouth to a target site within the patient's head via a transpalatine approach; and setting the orientation of the hollow tube relative to the mouthpiece by means of a tailor made mounting based on the patient's anatomy so that the hollow tube is in the desired orientation.

The method may comprise use of the guidance device of the first aspect as described above, and the guidance device used in this method may optionally have features as described in relation to the preferred/optional features of the first aspect. Herein disclosed is an exemplary method comprising targeting an intervention device as described above, and then carrying out the surgical intervention including surgically inserting the intervention device into the body and through the body tissue within the mouth to the target site in the patient's head.

The exemplary method may comprise use of a needle as the intervention device and optionally can include guided injection of a pharmacological substance into the body at the target site.

The mouthpiece may be attached to the patient's jaw by securing it to the patient's teeth. The mouthpiece may alternatively or additionally be mounted to the gums and/or the roof of the mouth. Using these parts of the patient's mouth for non-surgical attachment of the mouthpiece means that the mouthpiece can be attached by non-surgical personnel, or even by the patient themselves.

In another exemplary method may comprise navigated insertion of an intervention device toward the SPG along the transpalatine approach.

In a further exemplary method may comprise navigated insertion of an intervention device toward the OG.

The exemplary method may be for treating or preventing headache in a patient such as a human in need thereof and may comprise injecting a neuroinhibitory substance such as botulinium toxin in close proximity (i.e. proximally) to the sphenopalatine ganglion or otic ganglion wherein an intervention device in the form of an injection device comprising said neuroinhibitory substance is brought into close proximity to the sphenopalatine ganglion or otic ganglion by inserting said injection device into the patient via a transpalatine approach and the neuroinhibitory substance injected in close proximity to the SPG or OG.

The exemplary method may be for treating or preventing rhinitis, rhinosinusitis, Frey syndrome or hypersecretion of tears in a patient such as a human in need thereof and may comprise injecting a neuroinhibitory substance such as botulinium toxin in close proximity to the sphenopalatine ganglion or otic ganglion wherein an injection device comprising said neuroinhibitory substance is brought into close proximity to the sphenopalatine ganglion or otic ganglion by inserting said injection device into the patient via a transpalatine approach and the neuroinhibitory substance injected in close proximity to the SPG or OG.

The exemplary method may be for treating facial pain, including, orofacial pain disorders, myofascial pain disorders, temporomandibular joint disorders, neuropathic orofacial pain, trigeminal neuralgia and oral motor disorders.

Certain preferred embodiments will now be described by way of example only and with reference to the accompanying drawings in which:
Figure 1 shows a guidance device falling outside the scope of the present claims in a first perspective view;
Figure 2 shows the guidance device of Figure 1 in a second perspective view;
Figures 3a and b show the location of the SPG in the head with an intervention device shown approaching the SPG laterally;
Figure 4 shows the transnasal approach with an intervention device having an angled tip;
Figures 5a and b show a lateral approach to the OG;
Figure 6 shows a transnasal approach to the OG, this approach being defined by a straight line;
Figure 7 shows a guidance device in accordance with an embodiment of the invention in perspective view; and
Figure 8 shows the device of Figure 7 viewed from above.

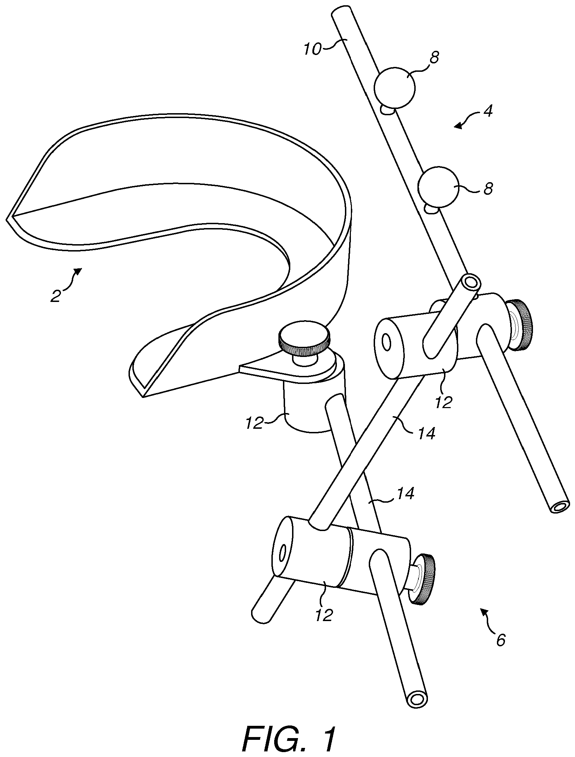

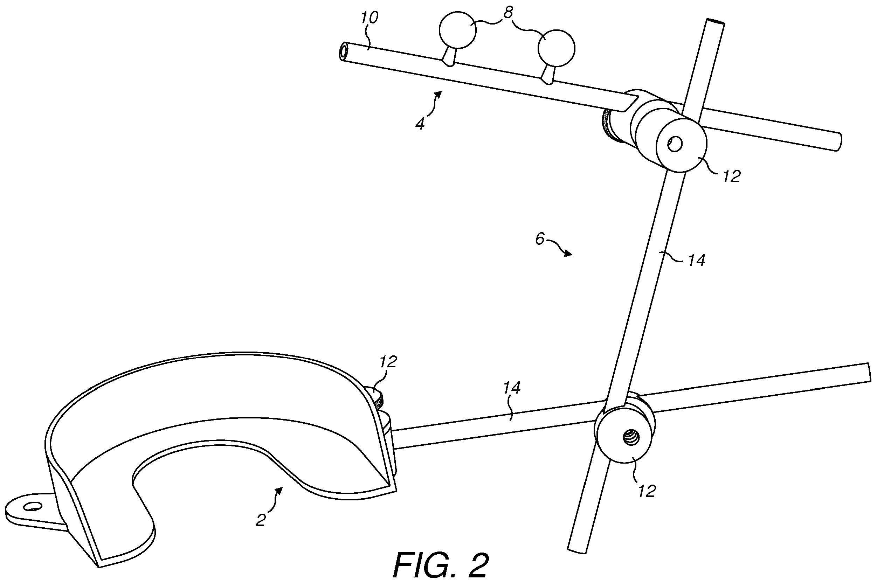

As shown in Figures 1 and 2 a guidance device is provided with a mouthpiece 2, a guide piece 4 and an adjustable coupling 6. The mouthpiece 2 in this arrangement has a shape similar to a gum shield, and can be attached to the patient's upper teeth via dental adhesive products, for example. The guide piece 4 has optical markers 8 forming a navigational array for use with an image guided navigation system. It will be understood that other forms of markers could be used. The guide piece 4 includes a lumen/guide tube 10 to which the navigational array 8 is attached. The lumen 10 can be aligned with a desired orientation so that an intervention device, such as a needle for example, can be inserted along the lumen toward a target site in the patient's head. The target site can be any location that is fixed relative to the upper jaw, for example it may be the SPG targeted via a lateral approach. The adjustable coupling 6 has clamps 12 that releasably attach the lumen 10 to the mouthpiece 2 via two rods 14. It will be understood that there may be differing numbers of clamps 12 and rods 14. Alternative designs of adjustable coupling 6 are also possible. Provided an adjustable and preferably lockable movement of the lumen 10 can be permitted then any type of joint can be used in the adjustable coupling 6.

With this arrangenent the mouthpiece is first fixed to the patient via the patient's teeth. Then the clamps 12 are loosened to permit a sliding and rotating motion of the rods 12 in order to allow adjustment of the lumen 10 into a desired orientation. Computer guided surgery systems can be used to guide the adjustment process. When the lumen 10 is at the desired angulation and location then the clamps 12 can be tightened to lock the mechanism. The interventionist may then carry out the required intervention with precision, since even if the patient moves the guide piece 4 will remain in the required orientation relative to the target site.

As described above, there may be a light source of a camera (not shown) attached to the device, preferably at the mouthpiece 2, in order to provide a visible alert of undesired movement of the guide piece 4 and/or mouthpiece 2 away from the desired orientation. The device can also include a screen (not shown) on a tablet or smart phone, with the screen being arranged to show guidance information from a computer navigation system.

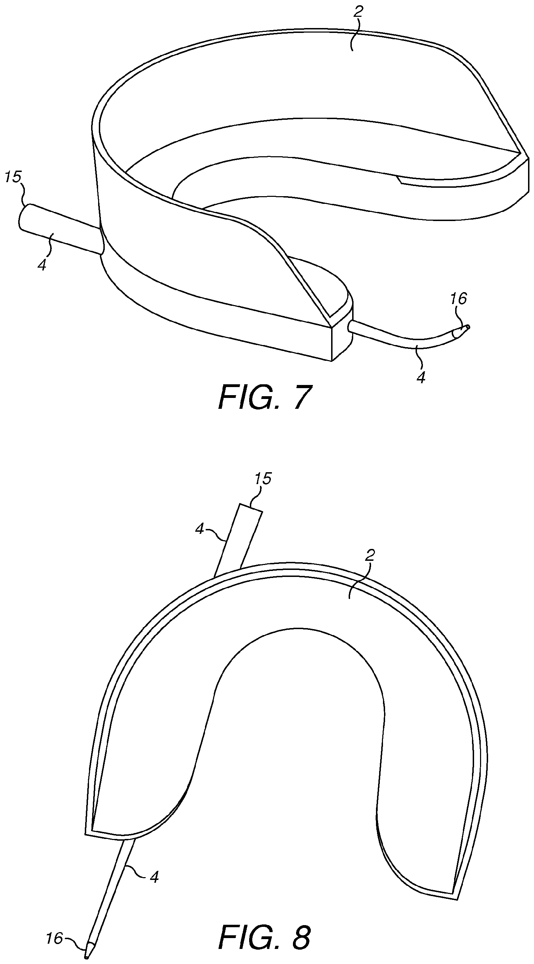

Figures 7 and 8 show a guidance device in accordance with an embodiment of the invention. This has a similar mouthpiece 2, but the guide piece 4 has a different design. This example uses a tailored guide piece 4, where the orientation of the guide piece 4 is set based on imaging data showing the patient's anatomy. In this example the guide piece 4 is arranged to target the SPG via a transpalatine approach. The mouthpiece 2 is for fixation to the patient's upper teeth to thereby fix the device (and the guide piece 4) relative to the upper jaw. The device is then fixed relative to the SPG and also to the transpalatine canal. The guide piece 4 has a proximal end 15 that will protrude out of the patient's mouth and can receive an intervention device, for example a needle. The guide piece 4 also has a distal end 16 that will be within the patient's mouth and has an orientation specifically tailored to the patient in order to direct the intervention device along the palatine canal toward the SPG.

It will be understood that either of the devices disclosed herein could be readily adapted for mounting to the lower teeth in order to hence allow for targeting of sites that are fixed relative to the lower jaw.

The proposed device leads to numerous advantages:
- It is not necessary to fixate the patient head and this enables procedures formerly performed under sedation/general anaesthesia to be performed on a conscious patient, leading to procedures with less complication and lower cost.
- The guide is attached to the patient without fixating the head, and will despite movement of the head point directly towards the target structure. The interventionist therefore can use both hands to handle the working device, syringe, pharmacological substances etc.
- The procedures will be much easier to perform and therefore within the reach of physicians and GPs, making the procedure more available to patients.
- The light source and/or camera will warn the interventionist if the device inadvertently moves relative to the target, making procedures more accurate and safer for the patient.
- The screen on device will make the image guided procedure more user-friendly and lower the threshold for performing the procedure for professionals not trained for conventional image guided procedure.

The devices described above makes it safer to use a lateral or transpalatine approach targeting the SPG, as well as other procedures (for example, targeting the OG), significantly lowering the risk of complications such as tissue destruction of adjacent structures by the very instrument at use or by adverse events due to misjudged placement of the needle while injecting the pharmacological substance. At the same time the positioning of the injection will be highly accurate, making it feasible to use small volumes with minimal possibilities of diffusion into adjacent structures. Such a precision also ensures optimal delivery of the pharmacological substances and therefore optimal treatment effect.

In the case of electromagnetic navigation, which can be used as an alternative or in addition to optical navigation, a coil can be embedded in the guide piece 4.

A possible advantageous use of the device is the injection of neuroinhibitory substances such as botulinum toxin in close proximity to the SPG or OG. Note that the injection device should not penetrate the SPG or OG. The injection is achieved in order to treat or prevent headache and may be achieved without damage to surrounding critical structures within the head. A neuroinhibitor is defined as any substance that affects transmission in a neural structure, resulting in any change of transmission, which may decrease or increase the neural activity. The neuroinhibitory substance is preferably a neurotoxin.

By delivery of the active substance in close proximity (proximally) to the sphenopalatine ganglion or otic ganglion means that the botulinum toxin or other neuroinhibitory substance in question is delivered so that it causes the desired technical effect, e.g. the prevention of treatment of headache etc. Ideally therefore the neuroinhibitory substance is injected to within 5 mm of the SPG or OG, preferably within 4 mm, such as within 3 mm, especially within 2 mm. Ideally injection of the active ingredient takes place 2 mm or less form the target SPG or OG.

The injection of the neuroinhibitor occurs laterally, transnasally, transorally or via the transpalatine approach in order to ensure that a safe, close injection of the neuroinhibitor is achieved. The terms laterally, infrazygomatically, transnasally and transorally are terms of this art.

The term infrazygomatic therefore requires that the injection takes place inferior to the zygomatic arch on either side of the mandibula, typically anterior or through the mandibular notch.

The term transnasally defines an injection route which involves advancing the needle through the nasal cavity. Targeting the SPG this route will further violate the lateroposterior boundary of the nasal cavity, constituting the medial boundary of the SF.

The preferred transoral approach is for the OG and hence includes advancing medial to the mandibular ramus and lateral to the midline of the head, in order to enter the infratemporal fossa and hence target the OG.

Targeting the OG transnasally involves advancing through the maxillary ostium and the maxillary sinus, violating the back wall of the maxillary sinus, advancing on the lateral aspect of the lateral pterygoid plate. The OG is located in the infratemporal fossa, the SPG in the sphenopalatine fossa.

It is preferably the case that access to the SPG or OG from the outside of the body is achieved laterally or transnasally by insertion of the injection device such that the device defines a straight line between SPG or OG (or more specifically the point proximal to the SPG and OG where active substance release will occur) and the point at which the external skin or mucosa is penetrated.

The lateral approach therefore allows the injection device to pass through the skin and then soft tissue to the SPG or OG. That can be achieved in a straight line and hence with a straight injection device. That means that the injection can be targeted very accurately in close proximity to the SPG or OG. This method of administration allows application under local anaesthetic.

Where the injection takes place transnasally the route involves passing through the nasal mucosa and the sphenopalatine foramen or the perpendicular plate of the palatine bone to reach the SPG. Injection is not therefore lateral (via the cheek) but preferably involves a straight line from the injection point to the SPG. Transnasal route to reach the OG involves advancing through the maxillary ostium and the maxillary sinus, violating the back wall of the maxillary sinus, advancing on the lateral aspect of the lateral pterygoid plate. This involves a straight line from the injection site to the OG. These methods may require general anaesthesia.

Using the transpalatine approach, as the guide piece 4 is accurately targeted toward the inferior opening of the palatine canal then high-precision interventions targeting the SPG are possible, unlike in the prior art. The oral cavity communicates with the sphenopalatine fossa through the greater traspalatinal canal. The inferior opening is situated on the medial side of the second molar and the length of the canal is on average 25 mm. The canal transmits the descending palatine artery and vein, and the greater and lesser palatine nerves.

The injection described above can be used in the treatment or prevention of headaches, in particular any kind of primary headache or secondary headache. The treatment or prevention may relate therefore to cluster headaches, migraine, tension-type headache, short lasting unilateral neuralgiform headache with conjunctival injection and tearing /cranial autonomic features (SUNCT/SUNA), hemicrania continua or paroxysmal hemicrania.

Paroxysmal hemicrania is a primary headache disorder involving frequent attacks of unilateral, peri-orbital and temporal pain typically lasting less than 30 minutes. The pain can be associated with conjunctival injection, lacrimation, nasal congestion, rhinorrhea, ptosis and eyelid edema.

SUNCT/SUNA is a primary headache disorder characterized by multiple attacks of unilateral, peri-orbital and temporal pain typically lasting less than 2 minutes. The pain is associated with conjunctival injection, lacrimation, nasal congestion, rhinorrhea, and eyelid edema. This headache may be associated with trigeminal neuralgia.

Hemicrania continua is a primary headache disorder characterized by a strictly unilateral headache responsive to Indomethacin. The pain is associated with conjunctival injection, lacrimation, nasal congestion, rhinorrhea, ptosis, and eyelid edema.

It will be appreciated that the term treatment here refers to reduction in pain experienced by a patient and/or a reduction in the frequency in which headache occurs. The term prevention means preventing headaches occurring, e.g. as frequently as before.

The neuroinhibitory substance is one which is capable of preventing or treating headache when administered in close proximity to the SPG or OG. Suitable inhibitors include Botulinum toxin, Tetanus neurotoxin, (which is produced by Clostridium tetani), Staphylococcal alpha-toxin, and acylpolyamine toxins (e.g. AR636 and AG489).

In general the therapeutic modality used to treat and/or prevent headache is a presynaptic neurotoxin. "Presynaptic neurotoxin" as used herein refers to those neurotoxins and their derivatives which are known to produce localized, reversible flaccid paralysis of musculature in mammals which does not result in degeneration of muscle or nervous tissue.

It is preferred however if the inhibitor is botulinum toxin. This is a protein and neurotoxin produced by the bacterium *Clostridium botulinum* and is commercially available. It is preferred if the botulinum toxin is of types A, B, C, D, E, F or G, such as Botulinum toxin type A. Botulinum toxin may for example be administered in the manner and form described in US 7981433

The frequency of the injections needed may be every 3 to 8 months but will be patient dependent.

Whilst the method described above is in relation to the administration of neuroinhibitory substances such as botulinium toxin, the method of injection and device discussed here can be used for the injection of other active substances such as local anaesthetics (e.g. lidocaine or marcain) and corticosteroids (e.g. triamcinolone). The method and device may be used to inject a local anaesthetic or corticosteroid for use in a method for treating or preventing headache, rhinitis, rhinosinusitis, Frey syndrome or hypersecretion of tears/lacrimation comprising injecting said substance in close proximity to the sphenopalatine ganglion or otic ganglion wherein an injection device comprising said substance is brought into close proximity to the sphenopalatine ganglion or otic ganglion by inserting said injection device into the patient transnasally or laterally and the substance injected in close proximity to the SPG or OG.

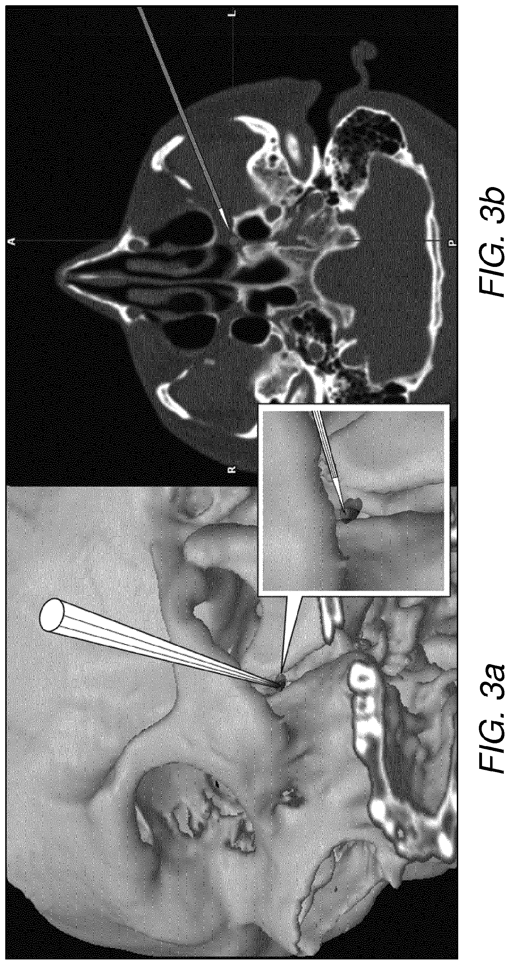

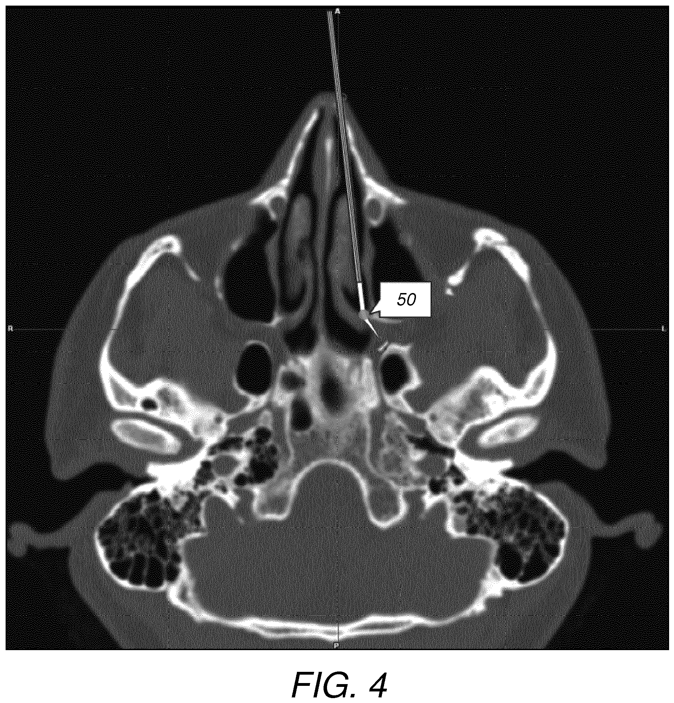

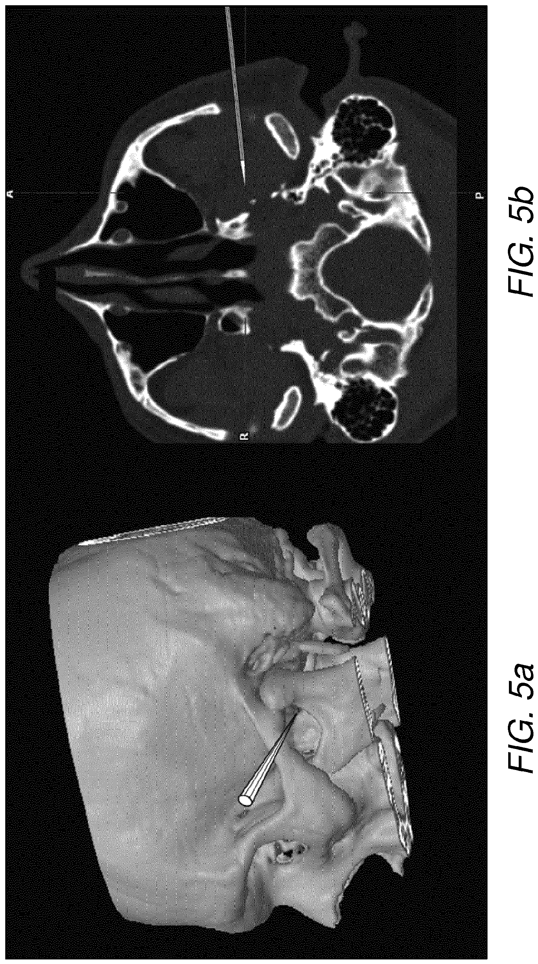

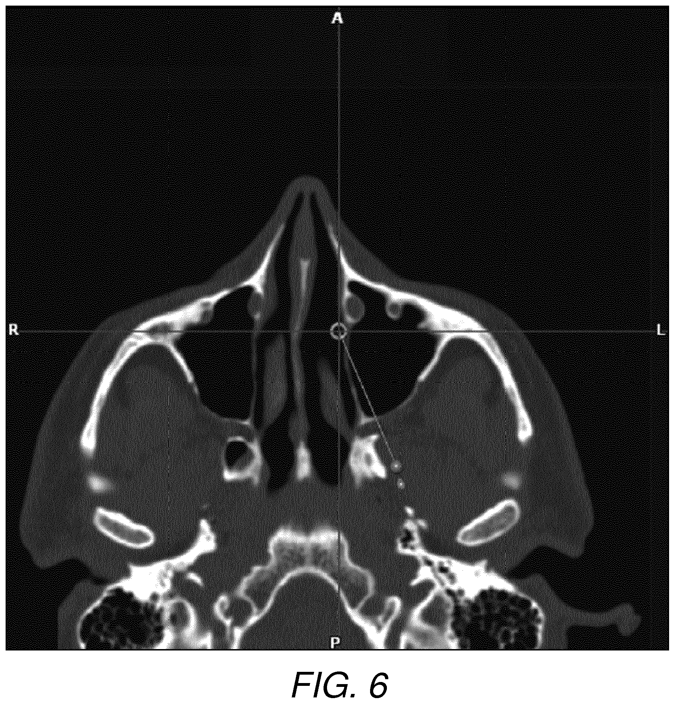

Various example procedures that can advantageously make use of the guidance device described above are set out below and Figures 3a through 6 illustrate the locations of the SPG and OG along with possible approaches for interventions on the SPG or OG as discussed above.

### Example 1

A female patient with refractory hemicrania continua was treated via injection of Botox around the SPG. Due to an occipital neurostimulator MRI was contraindicated and identification of SPG on MRI was not possible. Preoperatively the calculated position of the SPG was marked on a CT scan with 1 mm slides. On the navigation planning system a preplanned puncture site and trajectory was made. On the symptomatic side a navigable needle guide was advanced through the sphenopalatine foramen and towards the SPG. The needle was passed through the guide and the tip of the needle was confirmed to be 1 mm from the SPG by the navigation system while 75 IU botulinum toxin type A was injected.

Over a period of two months prior to the treatment the patient had an average headache intensity of 8.1 (scale 1-10) and normally experienced from one to four headache attacks daily. From 4 to 10 weeks after the treatment the patient had not a single attack during the whole period and the average headache intensity was 6.3. The patient also did not experience any complication during 4 months follow-up.

### Example 2

The patient was a male that presented with a prevertebral mass close to the atlas (C1) seen on MRI. He had formerly been treated for pulmonary cancer histologically classified as adenocarcinoma. After a clinical assessment it was concluded that the tumor was not available for conventional procedures for a histological diagnosis. Using a navigable guide with an optical navigation system and a transoral approach it was possible to do a fine needle biopsy of the tumor deep in the neck to confirm the suspicion of a pulmonary metastasis.

### Example 3

A female patient with refractory chronic cluster headache was treated via injection of lidocaine around the OG. Preoperatively the calculated position of the OG was marked on a CT scan with 1 mm slides. On the navigation planning system a pre-planned puncture site and trajectory was made. On the symptomatic side a navigable needle guide was advanced through the maxillary ostium and the back wall of the maxillary sinus, and then at the lateral aspects of the lateral pterygoid plate to the OG. 5ml of lidocaine 20mg/ml was injected. The patient had a short relief of the headache as expected using short-acting local anaesthetic.

### Example Applications

The advantages for interventions targeting the SPG will also arise when using the device for IGS in other parts of the patient that are fixed relative to the upper jaw for indications such as injections, biopsies, punctures, aspiration, ablation therapy, and for positioning of electrodes, catheters, radioactive seeds and implants. The design of the intervention device use with the guidance device can be varied as required. For example, it may be advantageous to use a similar device with an alternative tip design or a different length of end piece, depending on the characteristics of the target site, the approach available and the procedure that is to be carried out. The guidance device may thus be utilised for procedures to address numerous medical conditions. Procedures that the guidance device can be used for include:
- Injections
   ∘ Any pharmacological substance
   ∘ Neuroexcitatory agent
   ∘ Neuroinhibitory agents
   ∘ Botulinum toxin, any type
   ∘ Staphylococcal alpha-toxin
   ∘ Tetanus neurotoxin
   ∘ Acylpolyamine toxins
- Core needle biopsy and fine needle biopsy
   ∘ Head and neck area
   ▪ Intracranially
   ▪ Extracranially
      - Retropharyngeal space
      - Parapharyngeal space
      - Skull base
      - Deep regions of the face
      - Any region of the face
   ▪ In the vicinity of the columna
   ▪ In the vicinity of bone
- Puncture and aspiration
   ∘ Evacuation of cystic structures and fluidic compartment for diagnosis and therapy of the head and neck region
- Ablation therapy
   ∘ Any nerve or neural structure, intracranially and extracranially
   ∘ Ablation of normal tissue to reduce volume and/or increase stiffness
   ∘ Ablation of tumour tissue
- Positioning of electrodes, catheters, implants, electrophysiological measurements, radioactive seeds
- Endoscopy and/or pointer procedures
   ∘ Flexible or rigid endoscope may be attached to the device
   ∘ Any procedure in an open cavity that requires endoscope or pointer
      ▪ Paranasal sinusis
      ▪ Nasal cavity
      ▪ Farynx
      ▪ Larynx
- Facet blocks
   The device can be used in the treatment of conditions including:
- Headache
   ∘ Migraine
   ∘ Cluster headache
   ∘ Tension-type headache
   ∘ Trigeminal Autonomic Headache
   ∘ SUNCT
   ∘ Hemicrania Continua
   ∘ Paroxysmal hemicrania
   ∘ Any kind of primary headache
   ∘ Any kind of secondary headache
- Rhinitis
   ∘ Allergic rhinitis
   ∘ Vasomotor rhinitis
   ∘ Rhinitis medicamentosa
   ∘ Polypous rhinitis
   ∘ Any kind of non-structural rhinitis
- Rhinosinusitis
   ∘ Without polyps
   ∘ With polyps
   ∘ Any kind of rhinosinusitis
- Hypersecretion of tears/excessive lacrimation
   ∘ Any disease with hypersecretion of tears
- Frey syndrome/auriculotemporal syndrome/gustatory sweating
- Tinnitus
   ∘ Objective tinnitus
   ∘ Subjective tinnitus
- Neck and back pain/syndromes
   ∘ Spinal nerve/root blocks
   ∘ Spinal taps
- Post traumatic neck pain
- Cervical disc disorders
- Myofacial neck pain
- Rheumatic and arthritic disorders
- Neuromuscular disorders
- Facial pain
   ∘ orofacial pain disorders
   ∘ myofascial pain disorders
   ∘ temporomandibular joint disorders ∘ neuropathic orofacial pain
   ∘ trigeminal neuralgia
   ∘ oral motor disorders

Whilst the indications and examples above primarily relate to conditions of the human body the device can of course also be utilised for interventions on the animal body.

## Claims

1. A guidance device for guidance of surgical interventions on a patient, the surgical interventions requiring an intervention device to surgically enter the body and be directed through body tissues within the mouth to a target site within the patient's head, the guidance device comprising:
a hollow tube (4) for guiding the intervention device and directing it to the target site within the patient's head;
a mouthpiece (2) arranged to anchor the device in a fixed orientation relative to the patient's upper jaw or lower jaw; and
a targeted mounting supporting the hollow tube on the mouthpiece, the mounting being tailor-made for the patient based on the patient's anatomy and for directing the hollow tube in a desired orientation relative to the mouthpiece to thereby direct the intervention device through body tissues within the mouth to the target site in the patient's head via a transpalatine approach.

2. A device as claimed in claim 1, wherein the mouthpiece (2) is arranged to be mounted to the patient's upper or lower teeth in order to thereby anchor the device to the upper or lower jaw.

3. A device as claimed in claim 1 or 2, wherein the mouthpiece (2) is arranged to be mounted to the gums and/or the roof of the mouth; and/or
wherein the device further comprises a mouldable inner material for conforming to the patient's teeth, gums and/or roof of the mouth.

4. A device as claimed in any preceding claim, wherein the mounting is arranged for guiding the intervention device toward the SPG.

5. A device as claimed in any preceding claim, wherein the device includes an indicator for providing a warning of undesirable movement, preferably
wherein the device has a camera attached that can register an inadvertent movement.

6. A device as claimed in any preceding claim, comprising a patient localiser with a light source that can be directed towards a target on the mouthpiece.

7. A device as claimed in any preceding claim, comprising a proximal piece for holding a proximal end of an intervention device used with the guidance device, the proximal piece being arranged to allow for measurement of and/or control of the depth of insertion of an intervention device into the patient.

8. A device as claimed in any preceding claim, where the device is a single-use device intended to be disposed of after use.

9. A system for guided surgical interventions comprising a guidance device as claimed in any preceding claim along with an intervention device.

10. A system as claimed in claim 9, wherein the intervention device includes a navigation array, such as a localiser, in order to allow for guided placement of the intervention device, such as guided insertion to a required depth.

11. The system as claimed in claim 9 or 10, wherein the intervention device is a trackable needle that allows for the operator to track the location of the needle within the body, preferably wherein the intervention device is an electromagnetic needle.

12. A method of targeting an intervention device for later surgical intervention on the body by use of the guidance device or system of any of claims 1 to 11, the method comprising:
non-surgically attaching the mouthpiece (2) of the guidance device to the patient's upper jaw or lower jaw;
determining the desired orientation for the hollow tube (4) of the guidance device which will enable the hollow tube (4) to later guide the intervention device to surgically enter the body and be directed through body tissues within the mouth to the target site within the patient's head via a transpalatine approach; and
setting the orientation of the hollow tube (4) relative to the mouthpiece (2) by means of the tailor-made mounting based on the patient's anatomy so that the hollow tube is in the desired orientation.

13. A method as claimed in claim 12, wherein the hollow tube (4) in the desired orientation is oriented for guiding an intervention device toward the SPG.

## Patentansprüche

1. Führungsvorrichtung zur Führung von chirurgischen Interventionen an einem Patienten, wobei die chirurgischen Interventionen eine Interventionsvorrichtung erfordern, um chirurgisch in den Körper zu gelangen und durch Körpergewebe innerhalb des Mundes zu einer Zielstelle innerhalb des Kopfes des Patienten geleitet zu werden, wobei die Führungsvorrichtung umfasst:
eine Hohlröhre (4) zum Führen der Interventionsvorrichtung und Leiten zu der Zielstelle innerhalb des Kopfes des Patienten;
ein Mundstück (2), das angeordnet ist, um die Vorrichtung in einer festen Ausrichtung relativ zum Oberkiefer oder Unterkiefer des Patienten zu verankern; und
eine zielgerichtete Halterung, die die Hohlröhre an dem Mundstück stützt, wobei die Halterung basierend auf der Anatomie des Patienten maßgefertigt für den Patienten ist und dazu dient, die Hohlröhre in einer gewünschten Ausrichtung relativ zu dem Mundstück zu leiten, um dadurch die Interventionsvorrichtung über einen transpalatinen Ansatz durch Körpergewebe innerhalb des Mundes zu der Zielstelle in dem Kopf des Patienten zu leiten.

2. Vorrichtung nach Anspruch 1, wobei das Mundstück (2) angeordnet ist, um an den oberen oder unteren Zähnen des Patienten aufgebracht zu werden, um dadurch die Vorrichtung am Ober- oder Unterkiefer zu verankern.

3. Vorrichtung nach Anspruch 1 oder 2, wobei das Mundstück (2) angeordnet ist, um am Zahnfleisch und/oder am Gaumen aufgebracht zu werden; und/oder
wobei die Vorrichtung weiter ein formbares Innenmaterial umfasst, um dieses an die Zähne, das Zahnfleisch und/oder den Gaumen des Patienten anzupassen.

4. Vorrichtung nach einem der vorstehenden Ansprüche, wobei die Halterung angeordnet ist, um die Interventionsvorrichtung in Richtung des SPG zu führen.

5. Vorrichtung nach einem der vorstehenden Ansprüche, wobei die Vorrichtung eine Anzeige beinhaltet, um eine Warnung vor unerwünschter Bewegung bereitzustellen, vorzugsweise
wobei an der Vorrichtung eine Kamera angebracht ist, die eine unbeabsichtigte Bewegung registrieren kann.

6. Vorrichtung nach einem der vorstehenden Ansprüche, umfassend eine Patientenlokalisierungsvorrichtung mit einer Lichtquelle, die in Richtung eines Ziels an dem Mundstück gerichtet werden kann.

7. Vorrichtung nach einem der vorstehenden Ansprüche, umfassend ein proximales Stück zum Halten eines proximalen Endes einer Interventionsvorrichtung, die mit der Führungsvorrichtung verwendet wird, wobei das proximale Stück angeordnet ist, um eine Messung und/oder Steuerung der Tiefe der Einführung einer Interventionsvorrichtung in den Patienten zu ermöglichen.

8. Vorrichtung nach einem der vorstehenden Ansprüche, wobei die Vorrichtung eine Einwegvorrichtung ist, die dazu gedacht ist, nach dem Gebrauch entsorgt zu werden.

9. System für geführte chirurgische Interventionen, umfassend eine Führungsvorrichtung nach einem der vorstehenden Ansprüche zusammen mit einer Interventionsvorrichtung.

10. System nach Anspruch 9, wobei die Interventionsvorrichtung eine Navigationsanordnung beinhaltet, wie zum Beispiel eine Lokalisierungsvorrichtung, um geführte Platzierung der Interventionsvorrichtung, wie zum Beispiel geführte Einführung bis zu einer erforderlichen Tiefe, zu ermöglichen.

11. System nach Anspruch 9 oder 10, wobei die Interventionsvorrichtung eine verfolgbare Nadel ist, die dem Bediener ermöglicht, die Platzierung der Nadel innerhalb des Körpers zu verfolgen, wobei die Interventionsvorrichtung bevorzugt eine elektromagnetische Nadel ist.

12. Verfahren zum Anvisieren einer Interventionsvorrichtung für spätere chirurgische Intervention an dem Körper durch Verwendung der Führungsvorrichtung oder des Systems nach einem der Ansprüche 1 bis 11, wobei das Verfahren umfasst:
nicht-chirurgisches Anbringen des Mundstücks (2) der Führungsvorrichtung am Oberkiefer oder Unterkiefer des Patienten;
Bestimmen der gewünschten Ausrichtung für die Hohlröhre (4) der Führungsvorrichtung, wodurch ermöglicht wird, dass die Hohlröhre (4) die Interventionsvorrichtung später führt, sodass sie chirurgisch in den Körper gelangt und über einen transpalatinen Ansatz durch Körpergewebe innerhalb des Mundes zu der Zielstelle innerhalb des Kopfes des Patienten geleitet wird; und
Festlegen der Ausrichtung der Hohlröhre (4) relativ zu dem Mundstück (2) mittels der maßgefertigten Halterung basierend auf der Anatomie des Patienten, sodass sich die Hohlröhre in der gewünschten Ausrichtung befindet.

13. Verfahren nach Anspruch 12, wobei die Hohlröhre (4) in der gewünschten Ausrichtung ausgerichtet ist, um eine Interventionsvorrichtung in Richtung des SPG zu führen.

## Revendications

1. Dispositif de guidage pour guidage d'interventions chirurgicales sur un patient, les interventions chirurgicales nécessitant un dispositif d'intervention pour entrer chirurgicalement dans le corps et être dirigé à travers des tissus corporels dans la bouche jusqu'à un site cible dans la tête du patient, le dispositif de guidage comprenant :
un tube creux (4) pour guider le dispositif d'intervention et le diriger jusqu'au site cible dans la tête du patient ;
un embout buccal (2) agencé pour ancrer le dispositif dans une orientation fixe par rapport à la mâchoire supérieure ou à la mâchoire inférieure du patient ; et
une monture ciblée supportant le tube creux sur l'embout buccal, la monture étant faite sur mesure pour le patient sur la base de l'anatomie du patient et pour diriger le tube creux dans une orientation souhaitée par rapport à l'embout buccal afin de diriger ainsi le dispositif d'intervention à travers des tissus corporels dans la bouche jusqu'au site cible dans la tête du patient via une approche transpalatine.

2. Dispositif selon la revendication 1, dans lequel l'embout buccal (2) est agencé pour être monté sur les dents supérieures ou inférieures du patient afin d'ancrer ainsi le dispositif à la mâchoire supérieure ou inférieure.

3. Dispositif selon la revendication 1 ou 2, dans lequel l'embout buccal (2) est agencé pour être monté sur les gencives et/ou la voûte du palais ; et/ou
dans lequel le dispositif comprend en outre un matériau intérieur moulable pour se conformer aux dents, aux gencives et/ou à la voûte du palais du patient.

4. Dispositif selon l'une quelconque des revendications précédentes, dans lequel la monture est agencée pour guider le dispositif d'intervention vers le SPG.

5. Dispositif selon l'une quelconque des revendications précédentes, dans lequel le dispositif inclut un indicateur pour fournir un avertissement de déplacement indésirable, de préférence
dans lequel le dispositif comporte une caméra attachée qui peut enregistrer un déplacement accidentel.

6. Dispositif selon l'une quelconque des revendications précédentes, comprenant un localisateur de patient avec une source de lumière qui peut être dirigée vers une cible sur l'embout buccal.

7. Dispositif selon l'une quelconque des revendications précédentes, comprenant une pièce proximale pour maintenir une extrémité proximale d'un dispositif d'intervention utilisé avec le dispositif de guidage, la pièce proximale étant agencée pour permettre la mesure et/ou la commande de la profondeur d'insertion d'un dispositif d'intervention dans le patient.

8. Dispositif selon l'une quelconque des revendications précédentes, dans lequel le dispositif est un dispositif à usage unique destiné à être jeté après utilisation.

9. Système pour interventions chirurgicales guidées comprenant un dispositif de guidage selon l'une quelconque des revendications précédentes avec un dispositif d'intervention.

10. Système selon la revendication 9, dans lequel le dispositif d'intervention inclut un groupe de navigation, tel qu'un localisateur, afin de permettre un placement guidé du dispositif d'intervention, tel que l'insertion guidée à une profondeur nécessaire.

11. Système selon la revendication 9 ou 10, dans lequel le dispositif d'intervention est une aiguille traçable qui permet à l'opérateur de suivre l'emplacement de l'aiguille dans le corps, de préférence dans lequel le dispositif d'intervention est une aiguille électromagnétique.

12. Procédé de ciblage d'un dispositif d'intervention pour intervention chirurgicale ultérieure sur le corps par l'utilisation du dispositif de guidage ou du système selon l'une quelconque des revendications 1 à 11, le procédé comprenant :
la fixation non chirurgicale de l'embout buccal (2) du dispositif de guidage à la mâchoire supérieure ou à la mâchoire inférieure du patient ;
la détermination de l'orientation souhaitée pour le tube creux (4) du dispositif de guidage qui permettra au tube creux (4) de guider ultérieurement le dispositif d'intervention pour entrer chirurgicalement dans le corps et être dirigé à travers des tissus corporels dans la bouche jusqu'au site cible dans la tête du patient via une approche transpalatine ; et
le réglage de l'orientation du tube creux (4) par rapport à l'embout buccal (2) à l'aide de la monture faite sur mesure sur la base de l'anatomie du patient de telle sorte que le tube creux est dans l'orientation souhaitée.

13. Procédé selon la revendication 12, dans lequel le tube creux (4) dans l'orientation souhaitée est orienté pour guider un dispositif d'intervention vers le SPG.
